# EUROPEAN PATENT APPLICATION

(11) **EP 2 402 017 A1**
(43) Date of publication of application: **04.01.2012**
(21) Application number: 10746321.8
(22) Date of filing: 26.02.2010
(51) Int. Cl.: A61K 31/714, A61K 31/724, A61K 47/40, A61P 39/02, B01J 20/22, C01B 31/18

(54) **CARBON MONOXIDE REMOVAL AGENT**

(30) Priority: 26.02.2009 JP 2009043632
(71) Applicant: The Doshisha, Kyoto 602-8580 (JP); Tokai University Educational System, Tokyo 151-0063 (JP)
(72) Inventor: KANO Koji, Kyotanabe-shi Kyoto 610-0394 (JP); KITAGISHI Hiroaki, Kyotanabe-shi Kyoto 610-0394 (JP); NEGI Shigeru, Kyotanabe-shi Kyoto 610-0395 (JP); KIRIYAMA Akiko, Kyotanabe-shi Kyoto 610-0395 (JP); HONBO Akino, Kyotanabe-shi Kyoto 610-0395 (JP); KAWAGUCHI Akira, Isehara-shi Kanagawa 259-1193 (JP); TSUKADA Hideo, Hamamatsu-shi Shizuoka 435-8558 (JP)
(74) Representative: White, Duncan Rohan
(86) International application number: PCT/JP2010/053072
(87) International publication number: WO 2010/098442

(57) **Abstract**

The present invention provides a carbon monoxide removal agent that can be easily administered to a patient by injection or orally. The carbon monoxide removal agent of the present invention contains, as an active ingredient, an inclusion complex in which a cyclodextrin dimer represented by chemical formula (1) below includes a water-soluble metalloporphyrin. (In the formula, m represents either of number 1 or 2 and n represents any of number 1, 2, or 3.)

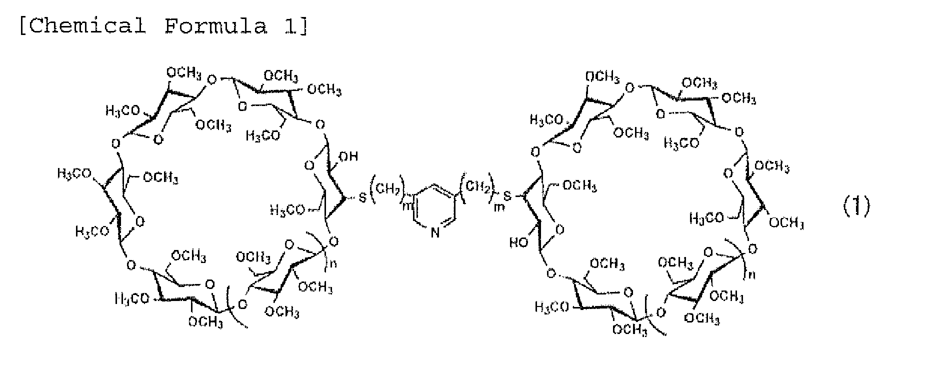

## Description

### TECHNICAL FIELD

The present invention relates to a carbon monoxide removal agent and particularly to a carbon monoxide removal agent that uses a porphyrin complex.

### BACKGROUND ART

Carbon monoxide (hereinafter abbreviated as "CO") is a toxic gas produced by incomplete combustion of carbon and when inhaled, it binds strongly with hemoglobin (hereinafter abbreviated as "Hb") in blood in place of oxygen (hereinafter abbreviated as "O₂"), depriving Hb of its inherent O₂ transport ability and causing an entire body to fall into an oxygen deprived state. Consequently, symptoms of so-called CO poisoning, such as headache, nausea, vomiting, physical deconditioning, confusion, loss of consciousness, chest pain, shortness of breath, coma, etc., occur.

The binding of Hb with O₂ or CO is reversible and an affinity of CO to Hb is approximately 250 times greater than that of O₂. Thus even when a small amount of CO is present in air, Hb is rapidly converted from an O₂ bound form to a CO bound form.

Presently, a CO poisoning remedy that reconverts the CO bound form of Hb to the O₂ bound form and thereby cure CO poisoning symptoms has yet to be developed. Thus as methods for recovery from poisoning, there were only methods of gradually shifting an equilibrium of Hb in blood from the CO bound form to the O₂ bound form by using a facemask to make high concentration oxygen be inhaled or by placing a CO poisoning patient under a high concentration O₂ atmosphere (see Non-Patent Document 1) .

However, these methods require certain facilities and in a large-scale fire or other case where a large number of CO patients occurs simultaneously, the patients cannot be treated efficiently and in some cases, this leads to deaths of patients. Also with these treatment methods, although a large part of the CO bound to Hb can be removed, it is difficult to remove CO that has spread widely to intricate parts of the body, and aftereffects of CO poisoning are a serious problem.

Meanwhile, the inventors have been conducting research from before on an inclusion complex formed by inclusion of a water-soluble metalloporphyrin by a cyclodextrin dimer, and have discovered that the inclusion complex has high affinities to O₂ and CO and that the affinity to CO is no less than 100 times that of Hb (see Patent Document 1, Non-Patent Document 2, and Non-Patent Document 3).

### PRIOR ART DOCUMENT(S)

### PATENT DOCUMENT(S)

Patent Document 1: Japanese Published Unexamined Patent Application No. 2006-2077

### NON-PATENT DOCUMENT(S)

Non-Patent Document 1: Satoshi Kitamura et al, "Manual of Emergency Treatments and Prescriptions for Carious Departments," pp.547-549, Ishiyaku Publishing Inc., 2005
Non-Patent Document 2: K. Kano et al, Angew. Chem. Int. Ed., 44, 435-438 (2005)
Non-Patent Document 3: K. Kano et al, Inorg. Chem. 45, 4448-4460 (2006)

### SUMMARY OF THE INVENTION

### PROBLEMS TO BE SOLVED BY THE INVENTION

Thus an object of the present invention is to provide a carbon monoxide removal agent that can be easily administered to a patient by injection or orally.

### MEANS FOR SOLVING THE PROBLEMS

The inventors noted that there is a possibility for the inclusion complex to be used as a carbon monoxide removal agent and have thereby come to complete present invention.

That is, a carbon monoxide removal agent according to a first aspect of the present invention contains, as an active ingredient, an inclusion complex in which a cyclodextrin dimer represented by chemical formula (1) includes a water-soluble metalloporphyrin. 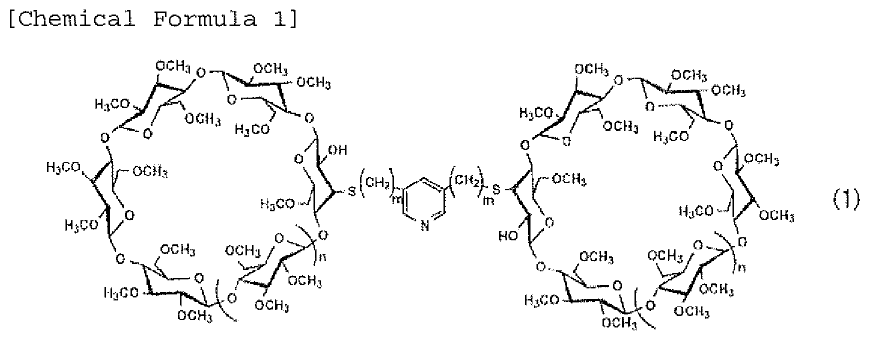 (In the formula, m represents either of number 1, or 2 and n represents any of number 1, 2, or 3.)

A carbon monoxide removal agent according to a second aspect is the carbon monoxide removal agent according to the first aspect where m=1 and n=2.

A carbon monoxide removal agent according to a third aspect is the carbon monoxide removal agent according to the first or second aspect where the water-soluble metalloporphyrin is represented by either of chemical formula (2) or (3). 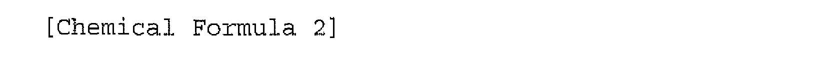 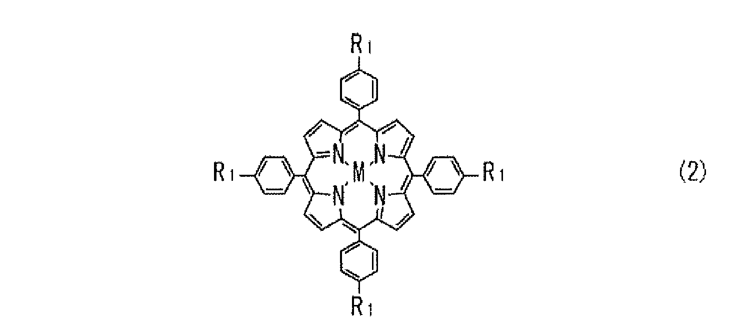 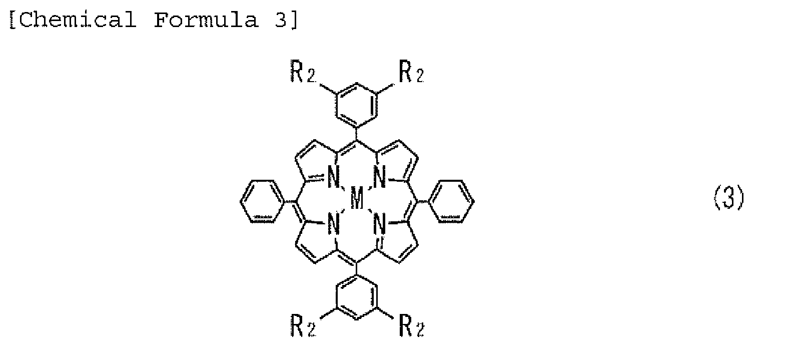 (In the formulae, each of R₁ and R₂ represents any of a carboxyl group, a sulfonyl group, or a hydroxyl group and M represents any of Fe²⁺, Mn²⁺, Co²⁺, or Zn²⁺.)

A carbon monoxide removal agent according to a fourth aspect is the carbon monoxide removal agent according to the third aspect where the water-soluble metalloporphyrin is 5,10,15,20-tetrakis(4-sulfonatophenyl)porphyrin (II) iron complex.

### EFFECT(S) OF THE INVENTION

The inclusion complex contained in the carbon monoxide removal agent according to the present invention has a higher affinity to CO than Hb and deprives Hb of CO contained in blood or peripheral tissue of a patient. The carbon monoxide removal agent according to the present invention is thus high in ability to treat COpoisoning. Thus, when the carbon monoxide removal agent according to the present invention is used clinically, many poisoning patients can be treated to save their lives.

The inclusion complex contained in the carbon monoxide removal agent according to the present invention can also absorb CO that is produced inside a body. Meanwhile, it is known that CO is produced by a decomposition reaction of hemoglobin and is related to biological reactions in functioning as a regulatory factor for gene expression, etc., (see, for example, S. Aono, Acc. Chem. Res., 36, 825-831 (2003)). The carbon monoxide removal agent according to the present invention can thus contribute not only to treatment of CO poisoning but also to research of such biological reactions.

### BRIEF DESCRIPTION OF THE DRAWINGS

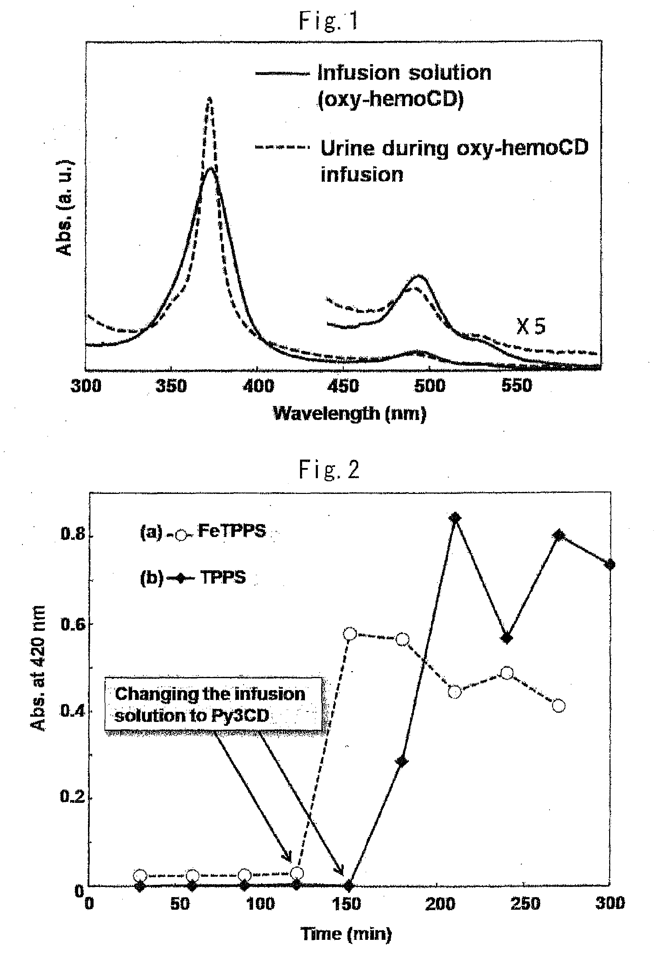

[FIG. 1] is a diagram comparing ultraviolet-visible absorption spectra of a carbon monoxide removal agent and excreted urine.
[FIG. 2] is a diagram showing change of absorbance at 420 nm of urine excreted upon continuously administrating a medical agent containing FeTPPS or TPPS to a rat.

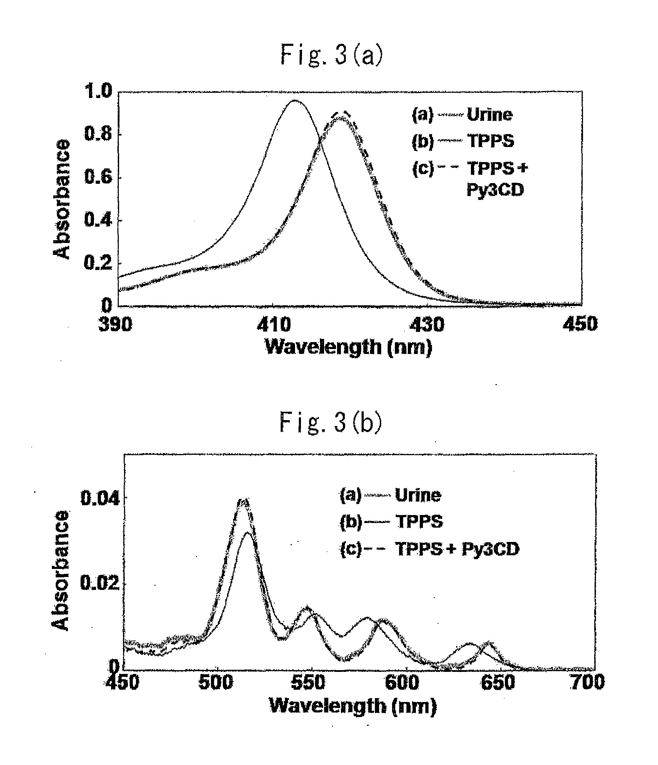

[FIG. 3] shows diagrams showing ultraviolet-visible absorption spectra of urine excreted upon continuously administrating a medical agent containing TPPS to a rat and thereafter starting administration of a Py3CD solution.

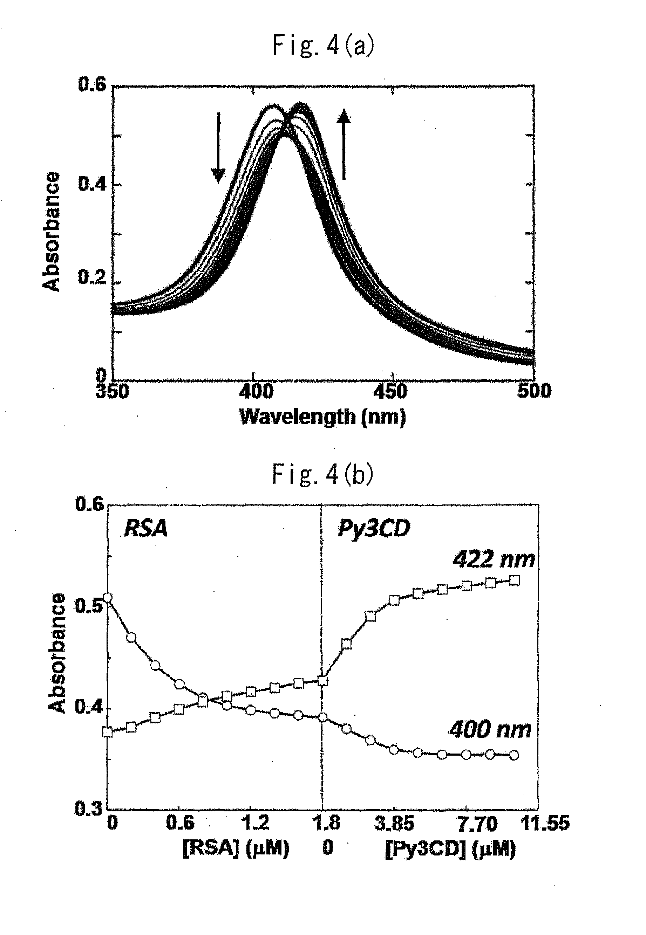

[FIG. 4] shows diagrams of results of measuring ultraviolet-visible absorption spectra while adding fixed amounts of an RSA solution to an FeTPPS solution and then from a point at which changes reached saturation, measuring ultraviolet-visible absorption spectra while adding a Py3CD solution in place of the RSA solution. FIG. 4 (a) is a diagram showing the changes in the ultraviolet-visible absorption spectra and FIG. 4(b) is a titration plot.

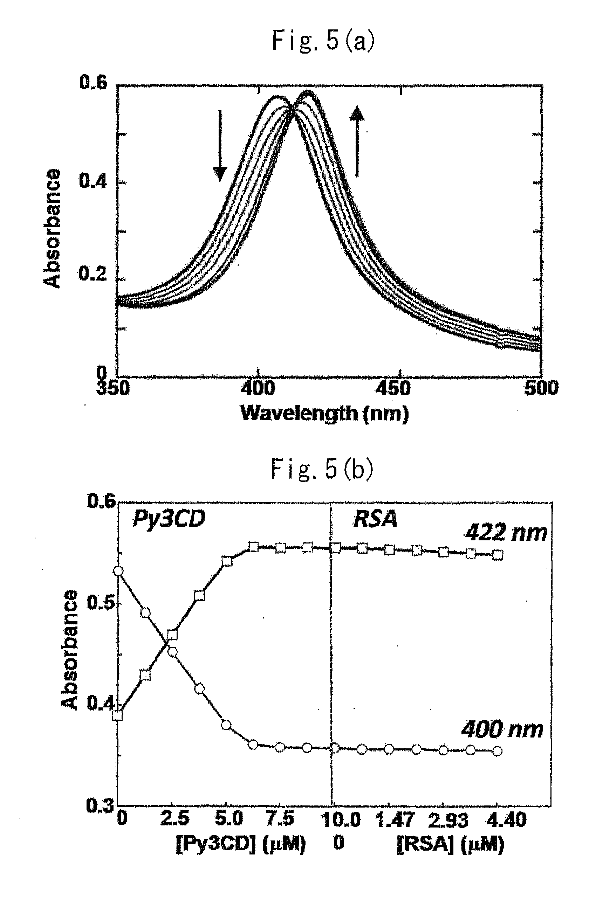

[FIG. 5] shows diagrams of results of measuring ultraviolet-visible absorption spectra while adding fixed amounts of the Py3CD solution to the FeTPPS solution and then from a point at which changes reached saturation, measuring ultraviolet-visible absorption spectra while adding the RSA solution in place of the Py3CD solution. FIG. 5 (a) is a diagram showing the changes in the ultraviolet-visible absorption spectra and FIG. 5(b) is a titration plot.

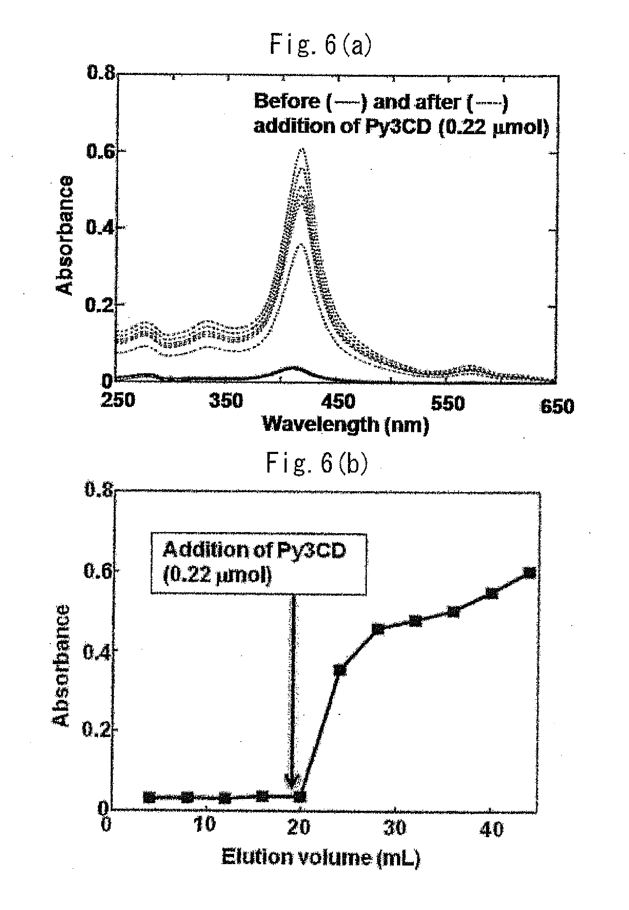

[FIG. 6] shows diagrams of results of examining a mechanism of excretion from blood into urine via kidneys using a kidney model that uses an ultrafiltration membrane. FIG. 6 (a) is a diagram showing changes in the ultraviolet-visible absorption spectra of filtrate and FIG. 6(b) is a diagram showing results of plotting change of absorbance at 420 nm against amount of filtrate.

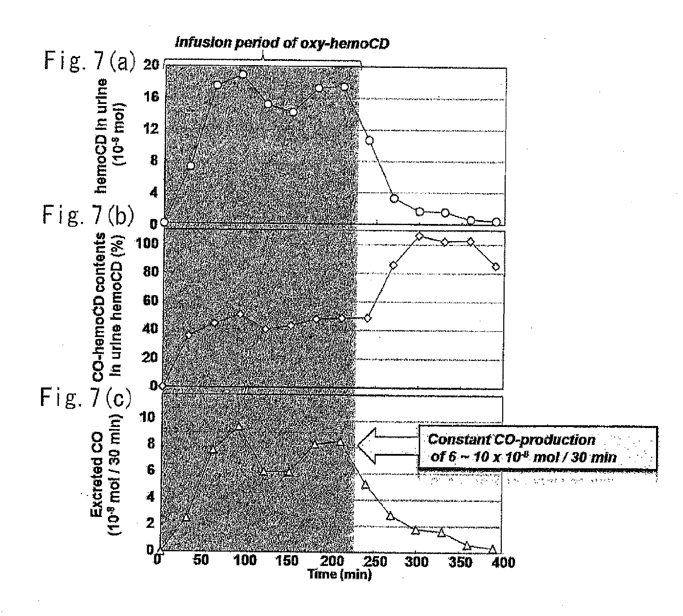

[FIG. 7] shows diagrams of results of examining changes with time of concentration of hemoCD excreted into urine, mol% of CO-hemoCD, and CO amount to examine dynamic states of a carbon monoxide removal agent within a body. FIG. 7(a) shows the concentration of hemoCD in urine, FIG. 7(b) shows the mol% of CO-hemoCD in hemoCD, and FIG. 7 (c) shows the excreted CO amount.

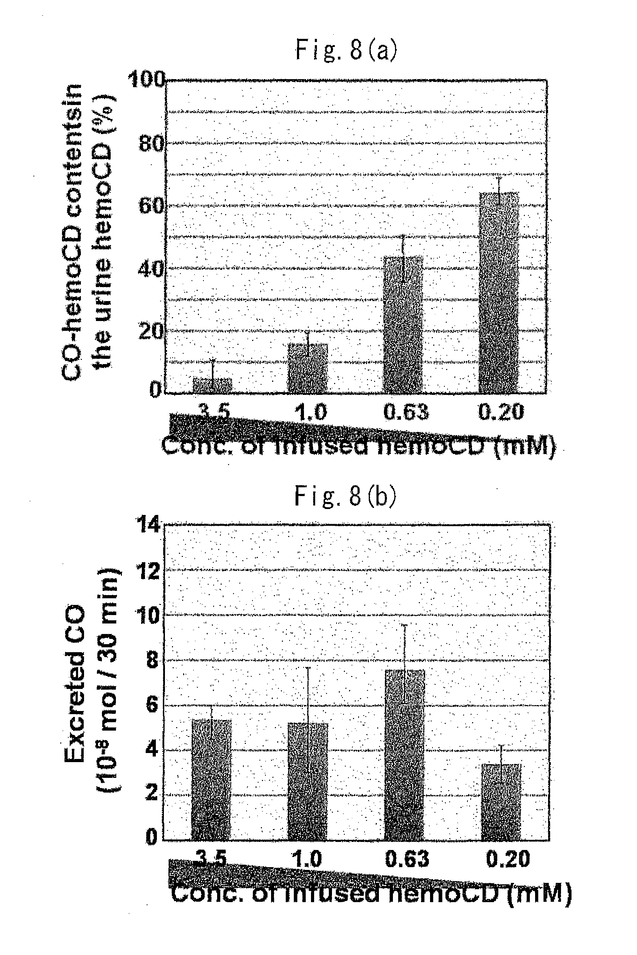

[FIG. 8] shows diagrams of results of examining influences of differences in the oxy-hemoCD concentration in the carbon monoxide removal agent on the mol% of CO-hemoCD in hemoCD contained in urine and the excreted CO amount. FIG. 8(a) is a diagram in which the mol% of CO-hemoCD is plotted against the oxy-hemoCD concentration, and FIG. 8(b) is a diagram in which the CO amount is plotted against the oxy-hemoCD concentration.

### MODE(S) FOR CARRYING OUT THE INVENTION

The carbon monoxide removal agent according to the present invention contains, as an active ingredient, an inclusion complex formed by inclusion of a water-soluble metalloporphyrin by a specific cyclodextrin dimer. The respective components shall now be described in detail. The inclusion complex can be manufactured by mixing the cyclodextrin dimer and the water-soluble metalloporphyrin in an aqueous solvent.

### 1. Cyclodextrin dimer

As shown in chemical formula (1), in the cyclodextrin dimer, two cyclodextrin molecules, with which all hydroxyl groups are methylated, are bound via 3,5-di(mercaptomethyl)pyridine, which is a linker molecule.

The cyclodextrin dimer is manufactured, for example, by tosylating and then epoxidizing cyclodextrin, thereafter methylating the hydroxyl groups of the cyclodextrin, and then binding the methylated cyclodextrins with the linker molecule as described in the prior art documents. The hydroxyl groups of cyclodextrin are methylated in advance to prevent difficulty of inclusion of the water-soluble metalloporphyrin in inner holes of the cyclodextrin dimer due to hardening of the inner holes of the cyclodextrins by hydrogen bonds formed by the hydroxyl groups.

The cyclodextrin that is the raw material of the cyclodextrin dimer is any of α-cyclodextrin, β-cyclodextrin, (m=1 and n=2), or γ-cyclodextrin, and among these, the use of β-cyclodextrins as the raw material is preferable because it readily includes the water-soluble metalloporphyrin.

### 2. Water-soluble metalloporphyrin

The water-soluble metalloporphyrin is a porphyrin-based compound that is voluble in water and has a metal ion coordinated at a center and is not restricted in particular as long as it can be included by the cyclodextrin dimer represented by the chemical formula (1).

Among water-soluble metalloporphyrins, a compound represented by the chemical formula (2) or the chemical formula (3) can be cited from a point of being reliably capable of adsorption and desorption of O₂, and more specifically, 5,10,15,20-tetrakis(4-sulfonatophenyl)porphyrin (II) iron complex (hereinafter abbreviated as "FeTPPS"), 5,15-bis(3,5-dicarboxylatophenyl)-10,20-diphenylporphyrin (II) iron complex (hereinafter abbreviated as "Fe-trans-2DC"), etc., can be cited. These compounds may, for example, be synthesized by a known method or a commercially available product (for example products of Frontier Scientific Inc., Tokyo Chemical Industry Co., Ltd., etc.) may be used as it is.

### 3. Dosage form, etc.

The carbon monoxide removal agent according to the present invention may be administered to a human or other animal in the form of the inclusion complex alone or by forming a medical composition with a known pharmaceutical carrier. A dosage form of the medical composition is not restricted in particular and may be selected appropriately as needed. Oral preparations, such as pills, capsules, granular agents, fine grain agents, powdered agents, and non-oral preparations, such as injectable agents, suppositories, embrocations, etc., can be cited as specific examples. A quantity of the carbon monoxide removal agent in the medical composition and a dosage amount of the medical composition for a patient may be selected freely according to the dosage form and age, weight, and degree of disorder of the patient.

In a case where the carbon monoxide removal agent according to the present invention is to be manufactured as a pill or other oral preparation, it can be manufactured by a known manufacturing method and using together a known diluent, binding agent, disintegrating agent, surfactant, lubricant, fluidity promoter, etc.

The carbon monoxide removal agent according to the present invention can also be orally administered as a suspension, an emulsion agent, a syrup agent, an elixir agent, etc. In this case, a flavoring agent, an odor improving agent, colorant, etc., may be contained.

In a case where the carbon monoxide removal agent according to the present invention is to be manufactured as a non-oral preparation, such as an inj ectable agent, drip, etc., it can be manufactured by a known manufacturing method and using together a known diluent, such as distilled water for injection, physiological saline diluent, glucose aqueous solution, etc. Also, a disinfectant, preservative, or stabilizer may be added as necessary. From a point of stability, the non-oral preparation may be frozen after being filled in a vial, etc., removed of water by an ordinary lyophilization process, and reconstituted as a liquid agent from the lyophilized product immediately before use. Further, atonicityagent, stabilizer, preservative, or soothing agent may be added as necessary.

As other examples of non-oral preparations of the carbonmonoxide removal agent according to the present invention, embrocations, such as liquid agents for external use, ointments, etc., suppositories for intrarectal administration, etc., can be cited, and these can also be manufactured according to known methods.

The carbon monoxide removal agent according to the present invention may be administered internally by a known DDS technique, for example, by sealing the carbon monoxide removal agent according to the present invention in a liposome or other carrier. In this case, by using a carrier that specifically recognizes cells of a target site, the carbon monoxide removal agent according to the present invention can be carried efficiently to the target site.

The present invention shall now be described in more detail based on examples. However, the scope of claims of the present invention is by no means restricted by the following examples.

### EXAMPLE 1

### 1. Confirmation of carbon monoxide removal ability

A carbon monoxide removal agent according to the present invention was prepared and a CO removal ability thereof was examined. Specifically, an experiment was performed as follows.

### (1) Reagents, etc.

As reagents, such as FeTPPS (made by Frontier Scientific Inc.), etc., commercially available products were used as they were. As Py3CD, that synthesized by the inventors according to Patent Document 1, Non-Patent Document 2, and Non-Patent Document 2 was used. As rats, Wister male rats (obtained from Shimizu Laboratory Supplies) were used. Ultraviolet-visible absorption spectra were measured using spectrophotometers (UV-2450 and MaltiSpec-1500, made by Shimadzu Corporation).

### (2) Preparation of the carbon monoxide removal agent

FeTPPS and Py3CD were respectively weighed out to a molar ratio of 1/1.2 using an electronic balance, placed in a beaker, and dissolved by adding 0.5 mL of a PBS buffer (pH 7.0) to the beaker. An excess amount (10 to 50 mg) of Na₂S₂O₄ was then added to the beaker to reduce the central iron in FeTPPS from Fe (III) to Fe(II).

The solution in the beaker was desalted by a HiTrap Desalting Column (made by GE Healthcare) (eluent: PBS buffer) to remove the excess Na₂S₂O₄. In this process, the reduced FeTPPS/Py3CD complex (hemoCD) becomes oxy-hemoCD because the Fe(II) that is the central ion binds with O₂ in air. After measuring the ultraviolet-visible absorption spectrum of the column-purified oxy-hemoCD solution to determine the concentration, the PBS buffer was used to adjust the solution to a predetermined concentration (0.2 to 3.5 mM) as the carbon monoxide removal agent.

### (3) Administration of the carbon monoxide removal agent to animal (rat) and aspiration of urine

A rat was put to sleep using a urethane anesthetic and a femoral region was exfoliated. Thereafter, the carbon monoxide removal agent was administered at a fixed rate (1.0 mL/h) from the femoral vein using a syringe pump. At every 30 minutes from the start of administration, urine was sampled from a vesicular portion, the interior of the bladder was then washed with physiological saline, and the urine and the physiological saline were put together and used as the urine in quantitative analysis (the same applies hereinafter).

### (4) Measurement of ultraviolet-visible absorption spectra

Ultraviolet-visible absorption spectra of the carbon monoxide removal agent prepared in (2) and the urine obtained in (3) were measured. The results are shown in FIG. 1. In FIG. 1, the solid line is the ultraviolet-visible absorption spectrum of the carbon monoxide removal agent and the dotted line is the spectrum of the urine.

From each of the ultraviolet-visible absorption spectra in FIG. 1, a peak wavelength (nm) and a full width at half maximum (FWHM (nm)) of a Soret absorption band, which is a characteristic absorption spectrum of a compound having a porphyrin ring, and a peak wavelength (nm) of a Q absorption band, which is also a characteristic absorption spectrum, were determined and these were compared. The results are indicated in Table 1.

**[Table 1]**

| | Soret (nm) [FWHM (nm)] | Q (nm) |
|---|---|---|
| oxy-hemoCD | 422 | 543 |
| | [33] | |
| CO-hemoCD | 422 | 540 |
| | [12] | |
| Urine | 422 | 540 |
| | [14] | |

### (5) Experimental results

From FIG. 1, it was found that between the carbon monoxide removal agent before administration and the urine, the ultraviolet-visible absorption spectrum, in particular, the peak of the Q absorption band changes (see the portion of X5 at the right side of FIG. 1). It was also found from the peak wavelength of the Q absorption band in Table 1 that the hemoCD in the carbon monoxide removal agent before administration is in the O₂ form (oxy-hemoCD) and the hemoCD in the urine is in the CO bound form (CO-hemoCD).

It is thus considered that when administered, the hemoCD exchanges the ligand from the original O₂ to CO, which is higher in affinity, in a process of being circulated through the entire body and is then excreted as urine. This suggests that there is a possibility for use of hemoCD as a carbon monoxide removal agent.

### EXAMPLE 2

### 2. Effect of the cyclodextrin dimer on the urinary excretion of porphyrins

The influence that the cyclodextrin dimer has on the excretion of the carbon monoxide removal agent in urine was examined. Specifically, each of the porphyrins was administered solitarily into a rat and absorbance at 420 nm, at which each administered porphyrin has a strong absorption band, was measured to examine the amount of the porphyrin excreted into urine. Also, after administration of each porphyrin, the cyclodextrin dimer was administered and the influence thereof was examined. Specifically, an experiment was performed as follows.

### (1) Preparation of medical agents, etc.

Commercially available FeTPPS (made by Frontier Scientific Inc.) and TPPS (made by Tokyo Chemical Industry Co. , Ltd.) with the central metal removed from FeTPPS were respectively dissolved separately to a concentration of 0.5 mM in a PBS buffer (pH 7.4) and thereby prepared as medical agents. Also, a Py3CD solution was prepared by dissolving Py3CD to a concentration of 0.6 mM in the PBS buffer (pH 7.4). Further, ultraviolet-visible absorption spectra were measured using the same apparatuses as in EXAMPLE 1.

### (2) Administration to animal and measurement of ultraviolet-visible absorption spectra

The medical agent containing FeTPPS was administered continuously to a rat by the syringe pump (1 mL/min). By the same method as in EXAMPLE 1, urine was sampled every 30 minutes from the start of administration and the change with time of the absorbance at 420 nm was examined. Also after 120 minutes from the start of administration of the medical agent, the administration of the medical agent containing FeTPPS was stopped, the Py3CD solution was administered by the syringe pump (1 mL/min) continuously for 180 minutes, urine was sampled every 30 minutes from the start of administration, and the change with time of the absorbance at 420 nm was examined. The results are shown in FIG. 2.

Also, the same experiment was performed using the medical agent containing TPPS. However, the administration of the Py3CD solution was started 150 minutes after the start of administration of the medical agent, and the administration time of the Py3CD solution was also 150 minutes. The results are also shown in FIG. 2.

Further, with the urine at the time of start of administration of the Py3CD solution among the urine sampled in the experiment using the medical agent containing TPPS, the ultraviolet-visible absorption spectrum was compared with the ultraviolet-visible absorption spectra of a separately prepared medical agent containing TPPS (2.5 µM) and a medical agent containing a TPPS (2.5 µM)/Py3CD (3.0 µM) complex. The results are shown in FIG. 3. Ultraviolet-visible absorption spectra from 390 nm to 450 nm are shown in FIG. 3(a), and ultraviolet-visible absorption spectra from 450 nm to 700 nm are shown in FIG. 3(b).

### (3) Experimental results

From FIG. 2, it was found that even when the medical agent containing just FeTPPS or the medical agent containing just TPPS is administered, FeTPPS or TPPS is not excreted at all in urine. Also from the figure, it was found that the excretion of FeTPPS or TPPS in urine is initiate by administration of Py3CD. From these results, it was confirmed that the administration of Py3CD induces the excretion of FeTPPS or TPPS in urine.

Also from FIG. 3, it was found that the shape of the ultraviolet-visible absorption spectrum of the urine matches that of the TPPS/Py3CD complex well. It is thus considered that even though only TPPS was administered into the animal, the TPPS excreted after administration of Py3CD is included in the Py3CD. It was thus confirmed that Py3CD includes TPPS inside a living body as it does in an in vitro system and that the formation of the inclusion complex induces excretion.

### EXAMPLE 3

### 3. Comparison of urinary excretion induction effect

Serum albumin (RSA) is a protein component of the highest content in blood and is known to have a property of taking in various hydrophobic molecules. FeTPPS is not an exception and is known to bind strongly to serum albumin (see, for example, V. E. Yushmanov et al, Mag. Res. Imaging, 14, 255-261 (1996), T. T. Tominaga et al, J. Inorg. Biochem., 65, 235-244 (1997), etc.). The effect of inducing urinary excretion of FeTPPS by Py3CD was thus compared with that by RSA. Specifically, an experiment was performed as follows.

### (1) Preparation of medical agents, etc.

FeTPPS was dissolved in a PBS buffer (pH 7.4) to prepare a 5 µM solution, and Py3CD and RSA (made by SIGMA Co., Ltd.) were separately dissolved in the PBS buffer (pH 7.4) to prepare respective stock solutions. Ultraviolet-visible absorption spectra were measured with the same apparatuses as those used in EXAMPLE 1.

### (2) Measurements of ultraviolet-visible absorption spectra

The FeTPPS solution was placed in a cuvette, ultraviolet-visible absorption spectra were measured while adding fixed amounts of the RSA solution to the cuvette, and at a point at which changes reached saturation, ultraviolet-visible absorption spectra were measured while adding the Py3CD solution in place of the RSA solution. The results are shown in FIG. 4. FIG. 4(a) is a diagram showing the changes in the ultraviolet-visible absorption spectra and FIG. 4(b) is a titration plot.

Oppositely, the FeTPPS solution was placed in a cuvette, ultraviolet-visible absorption spectra were measured while adding fixed amounts of the Py3CD solution to the cuvette, and at a point at which changes reached saturation, ultraviolet-visible absorption spectra were measured while adding the RSA solution in place of the Py3CD solution. The results are shown in FIG. 5. FIG. 5(a) is a diagram showing the changes in the ultraviolet-visible absorption spectra and FIG. 5(b) is a titration plot.

### (3) Experimental results

From FIG. 4 (a), it was found that the ultraviolet-visible absorption spectral changes as RSA is added. Also from FIG. 4 (b), it was found that the changes in the ultraviolet-visible absorption spectra reach saturation at the point at which RSA of approximately 1/3rd the equivalent amount with respect to the concentration (5×10⁻⁶ M) of FeTPPS is added. This suggests that approximately three FeTPPS molecules can bind to a single RSA molecule.

Further, from FIG. 4(b), it was found that when Py3CD is added after saturation by the RSA solution, the ultraviolet-visible absorption spectra exhibit stepwise changes again and that the changes saturate at the point at which Py3CD of an equivalent amount with respect to FeTPPS is added. In addition, it was found that the peak of the final ultraviolet-visible absorption spectrum is 422 nm and matches the peak of the FeTPPS/Py3CD complex. These results suggest that FeTPPS changes from an RSA bound form to a Py3CD bound form and that Py3CD is higher in the affinity to FeTPPS than RSA.

Meanwhile, from FIG. 5(a), it was found that the ultraviolet-visible absorption spectral changes even when Py3CD is added. Also from FIG. 5(b), it was found that even when RSA is added after FeTPPS is saturated by the addition of Py3CD, the ultraviolet-visible absorption spectrum does not change. This suggests that Py3CD is higher in the affinity to FeTPPS than RSA.

### EXAMPLE 4

### 4. Examinationof the mechanism of excretion of the carbon monoxide removal agent

The mechanism by which the carbon monoxide removal agent according to the present invention is excreted from blood into urine via kidneys was examined by way of a kidney model using an ultrafiltration membrane. Specifically, an experiment was performed as follows.

### (1) Preparation of medical agents, etc.

FeTPPS, RSA, and Py3CD (procured from the same firms as in EXAMPLE 3) were respectively dissolved in a PBS buffer (pH 7.4) to prepare 0.22 µM solutions. Also, ultraviolet-visible absorption spectra were measured using the same apparatuses as in EXAMPLE 1. As the ultrafiltration membrane, a stirred cell (Model 8050 made by Millipore Corp.) withanultrafiltration membrane with a molecular weight cutoff of 30,000 attached was used.

### (2) Diafiltration test

Equivalent amounts of the FeTPPS solution and the RSA solution were mixed to prepare an equimolar mixture, and the equimolar mixture solution was placed in the stirred cell that was set above a recovery beaker. While pressurizing with nitrogen gas, a filtrate was recovered at every fixed amount and subject to ultraviolet-visible absorption spectral analysis.

At a point at which 20 mL of the filtrate was recovered, the Py3CD solution was added so that the molar amount of Py3CD was equal to the molar amount of each of FeTPPS and RSA in the residual solution. Thereafter, while pressurizing with nitrogen gas, a filtrate was recovered at every fixed amount and subject to ultraviolet-visible absorption spectral analysis. The results are shown in FIG. 6. FIG. 6(a) shows the ultraviolet-visible absorption spectra and FIG. 6 (b) shows results of plotting change of absorbance at 420 nm against the filtrate amount.

### (3) Experimental results

From FIG. 6(a) and 6(b), it was found that until the Py3CD solution is added, the ultraviolet-visible absorption spectrum scarcely changes, and from the point at which the Py3CD solution is added, the ultraviolet-visible absorption spectrum, in particular, the absorbance at 420 nm wavelength changes greatly as the filtrate amount increases.

The molecular weight of RSA is 68,000 and the molecular weight of Py3CD is approximately 3,000. The experimental results thus suggest that whereas while FeTPPS is held by RSA, it cannot pass through the ultrafiltration membrane, when FeTPPS is released from RSA by addition of Py3CD and becomes included in Py3CD, it becomes capable of passing through the ultrafiltration membrane.

In the same manner as in the principle of ultrafiltration, filtration membranes of the kidneys also perform screening according to molecular size and serum albumin is not excreted in urine. In consideration of this and the above experimental results, it is considered that the FeTPPS excretion induction effect of Py3CD is due to a change of molecular size.

### EXAMPLE 5

### 5. Examination of dynamics of the carbon monoxide removal agent

To examine the dynamics of the carbon monoxide removal agent inside the body, changes with time of the concentration of hemoCD excreted into urine, mol% of CO-hemoCD in hemoCD, and CO amount were examined. Specifically, an experiment was performed as follows.

### (1) Preparation of medical agents, etc.

An oxy-hemoCD solution was prepared by the method described for EXAMPLE 1 and used as the carbon monoxide removal agent. Procurement of the experimental animal (rat), administration of the medical agent, sampling of urine, and ultraviolet-visible absorption spectral analysis were performed in the same manner as in EXAMPLE 1.

### (2) Progressive changes of hemoCD concentration in urine, mol% of CO-hemoCD in hemoCD, and CO amount

Urine was sampled every 30 minutes from the start of administration of the medical agent, the ultraviolet-visible absorption spectra of the urine were measured, and the hemoCD concentration in urine, the mol% of CO-hemoCD in hemoCD, and the CO amount excreted into urine were computed. The results are shown in FIG. 7. FIG. 7 (a) shows the hemoCD concentration, FIG. 7 (b) shows the mol% of CO-hemoCD in hemoCD, and FIG. 7 (c) shows the excreted CO amount. The hemoCD concentration in urine, the mol% of CO-hemoCD in hemoCD, and the CO amount in urine were computed from the absorbance as follows.

The molar concentration of hemoCD in the sampled urine was determined by adding suitable amounts of Na₂S₂O₄ and CO gas to the urine to convert all of the hemoCD in the urine to the CO bound form, measuring the ultraviolet-visible absorption spectrum of the converted urine to determine the absorbance at the maximum absorption wavelength, and computing the concentration from the absorbance and the molar extinction coefficient indicated in the prior art documents, etc.

The mol% of CO-hemoCD in hemoCD contained in the sampled urine was determined by measuring the ultraviolet-visible absorption spectra of the urine as it is and the urine with all of the hemoCD therein converted to the CO bound form and computing the concentration from the absorbance difference at the maximum absorption wavelength. Also, the CO amount in urine was computed from the molar concentration of hemoCD contained in urine, the mol% of CO-hemoCD in hemoCD, and the urine amount.

### (3) Experimental results

FIG. 7(a) shows that the excretion of hemoCD starts immediately after the start of administration and the amount of excreted hemoCD decreases extremely at nearly the same time as the end of administration. These suggest that the internal retention time of hemoCD is considerably short. Also from FIG. 7 (b), it was found that when hemoCD is being excreted at a large amount, the mol% of CO-hemoCD is low and when the excretion amount of hemoCD is low, the mol% of CO-hemoCD is high. With FIG. 7(c), when the CO amount is converted to the number of moles of CO excreted and this is plotted, it was found that CO is excreted at a substantially fixed rate of within 6 to 10×10⁻⁸ mol/30 min during the time in which a sufficient amount of hemoCD is administered.

### EXAMPLE 6

### 6. Influences of differences in administered hemoCD amount on CO excretion rate, etc.

Influences of differences in the concentration of oxy-hemoCD in the carbon monoxide removal agent administered on the mol% of CO-hemoCD in the hemoCD excreted into urine and the amount of CO excreted into urine were examined. Specifically, the following was performed.

### (1) Experimental method

After preparing carbon monoxide removal agents of different oxy-hemoCD concentrations, the same experiment as in EXAMPLE 5 was performed to compute the mol% of CO-hemoCD in hemoCD and the excreted CO amount. The results are shown in FIG. 8. FIG. 8(a) shows the results of plotting the mol% of CO-hemoCD in hemoCD versus the oxy-hemoCD concentration in the carbon monoxide removal agent, and FIG. 8(b) shows the results of plotting the excreted CO amount versus the oxy-hemoCD concentration in the carbon monoxide removal agent.

### (2) Experimental results

From FIG. 8 (a), it was found that when the concentration of the administered hemoCD is changed, the mol% taken up by the CO-hemoCD changes according to the administration concentration. However from FIG. 8(b), it was found that the CO excretion amount per unit time computed from the above result does not change. That is, while CO is being removed from inside the body by hemoCD, CO production occurs at a pace of 3 to 10×10⁻⁸ mol/30 min inside the body, suggesting an action in a direction of maintaining the CO concentration inside the body.

## Claims

1. A carbon monoxide removal agent comprising, as an active ingredient, an inclusion complex in which a cyclodextrin dimer represented by chemical formula (1) includes a water-soluble metalloporphyrin. 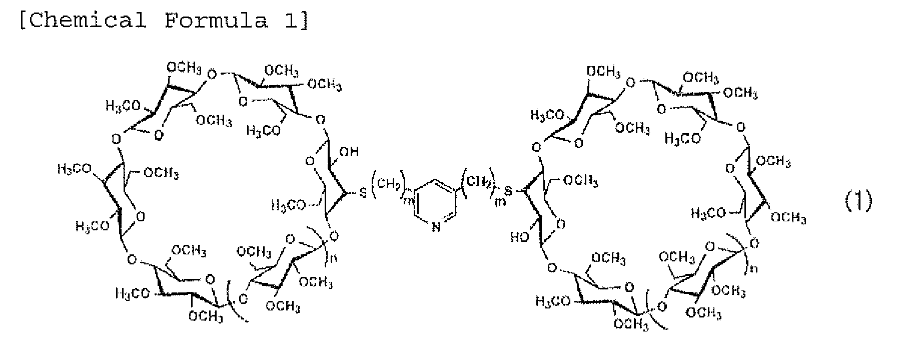 (In the formula, m represents either of number 1 or 2 and n represents any of number 1, 2, or 3.)

2. The carbon monoxide removal agent according to Claim 1, wherein m=1 and n=2.

3. The carbon monoxide removal agent according to Claim 1 or 2, wherein the water-soluble metalloporphyrin is represented by either of chemical formula (2) or (3). 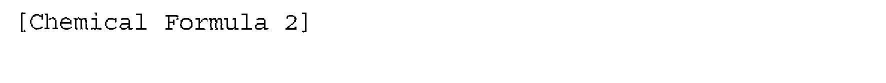 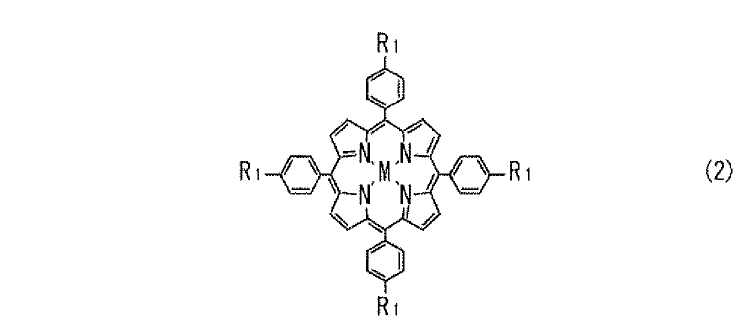 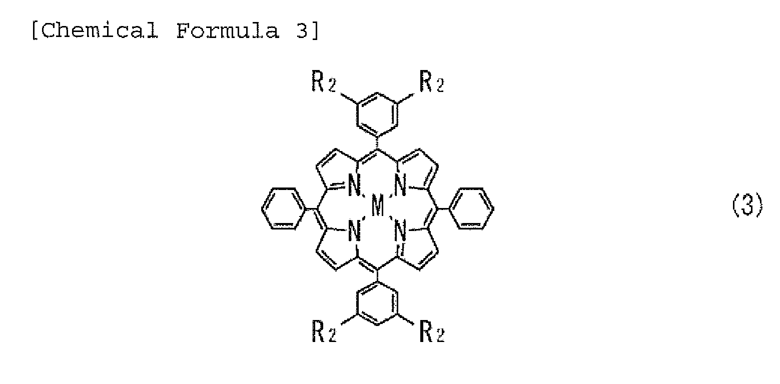 (In the formulae, each of R₁ and R₂ represents any of a carboxyl group, a sulfonyl group, or a hydroxyl group and M represents any of Fe²⁺, Mn²⁺, Co²⁺, or Zn²⁺.)

4. The carbon monoxide removal agent according to Claim 3, wherein the water-soluble metalloporphyrin is 5,10,15,20-tetrakis(4-sulfonatophenyl)porphyrin (II) iron complex.
